Europäisches Patentamt

European Patent Office

Office européen des brevets

(19)

(11) Numéro de publication : **0 454 600 A1**

# DEMANDE DE BREVET EUROPEEN

(12)

(21) Numéro de dépôt : 91420138.9

(22) Date de dépôt : 25.04.91

(51) Int. Cl.⁵ : **C07K 15/20, A61K 37/12, C12N 5/00, A61L 15/32**

(30) Priorité : 25.04.90 FR 9005941

(43) Date de publication de la demande :
**30.10.91 Bulletin 91/44**

(84) Etats contractants désignés :
**AT BE CH DE DK ES GB GR IT LI LU NL SE**

(71) Demandeur : **ICP FRANCE, Société Anonyme**
**Zac Val Varinot B.P. No. 91 Boulevard**
**Maréchal Juin**
**F-52003 Chaumont Cédex (FR)**
Demandeur : **Michel, Jean-Pierre**
**Rochetaillée**
**F-52210 Arc-en-Barrois (FR)**

(72) Inventeur : **Gagnieu, Christian**
**19 Rue Pierre et Marie Curie**
**F-69680 Chassieu (FR)**
Inventeur : **Valherie, Isabelle**
**11 Bis Cours de la République**
**F-69100 Villeurbanne (FR)**

(74) Mandataire : **Dupuis, François et al**
**Cabinet Laurent et Charras, 3 Place de**
**l'Hôtel-de-Ville, BP 203**
**F-42005 St. Etienne Cédex 1 (FR)**

(54) **Produit à base de kératines modifiées, son procédé de préparation et les applications notamment en médecine humaine ou vétérinaire.**

㊗ Le produit à base de kératines modifiées est
une S-aminoAlkylkératines de fomule générale :
$$R - S - (CH_2)_n - NH_2$$
dans laquelle :
— n est un nombre entier égale à 2 ou 3
— R représente la kératine

EP 0 454 600 A1

Plus particulièrement, mais non limitativement, l'invention s'est fixée pour but de créer un produit trouvant une application avantageuse :

– pour le recouvrement et la protection des plaies étendues telles que brûlures, pertes de substances...

– pour le recouvrement et la protection des greffes cutanées notamment, les greffes de tissus vivants ou non, les greffes de dermes artificiels ou de peaux artificielles,

– en tant que support et moyen de protection de feuillets épidermiques cultivés pour les greffes d'épiderme.

Actuellement, les produits utilisés pour les applications précitées, ne donnent pas entière satisfaction. Généralement, on utilise soit de la gaze, soit des matériaux à base de silicones sous différentes formes, soit des tissus synthétiques microporeux.

La gaze, le plus souvent imprégnée de vaseline, a pour avantage de limiter les pertes hydriques, salines, et calorico-azotées, et assure une protection vis à vis des contaminations bactériennes. Par contre, un tel pansement a une perméabilité à l'eau non contrôlable, ce qui peut engendrer un effet de macération préjudiciable à la cicatrisation. De plus, ce type de pansement doit être changé fréquemment.

L'utilisation de la gaze pour le transfert de cellules épidermiques cultivées est limitée par le fait que lors de la régénération de l'épiderme, les cellules peuvent migrer et se multiplier à l'intérieur de la gaze, ce qui entraine une incrustation de ce matériau dans le tissu néoformé.

Les matériaux à base de silicones appliqués sous forme de mousses ou de films, ne permettent pas de controler les pertes hydriques et des phénomènes de macération peuvent également se manifester comme dans le cas de la gaze imprégnée de vaseline.

Enfin, les tissus microporeux, utilisés pour les greffes de cellules épidermiques présentent l'inconvénient de ne pouvoir être facilement retirés du site de cicatrisation en raison de la prolifération des cellules à l'intérieur du matériau.

On connait également des produits à base de kératines.

Dans le brevet FR 2522657 les kératines totales sont extraites de la plume de volailles. Ces molécules de kératines ne sont pas purifiées et contiennent de ce fait, des protéines non kératéniques. Les kératines extraites des plumes ont une structure en "feuillet β" qui leur confère des propriétés mécaniques très particulières.

En outre, les produits décrits dans ce brevet, sont tous solubles dans l'eau et gardent cette propriété après avoir été déshydratés.

Dans le brevet FR 2529214 les produits obtenus ont des caractéristiques physiques et physicochimiques telles qu'ils sont solubles dans l'eau, et ne présente plus la structure native des kératines puisqu'ils sont obtenus à partir de fragments de kératines.

L'invention s'est fixée pour but de remédier à ces inconvénients.

Un des problèmes que se propose de résoudre l'invention est d'obtenir un produit soluble dans l'eau, avant déshydratation et apte à devenir insoluble après déshydratation.

Un tel problème est résolu en ce qu'il a été conçu selon l'invention, un produit à base de kératines modifiées sous forme d'une S-aminoAlkylkératines de fomule générale :

$$R - S - (CH_2) n - NH_2$$

dans laquelle :

– n est un nombre entier égale à 2 ou 3

– R représente la kératine

Avantageusement, le produit est obtenu à partir d'une solution de S-aminoéthylkératines de formule:

$$R - S - CH_2 - CH_2 - NH_2$$

obtenues par réduction puis S-alkylation des kératines réduites avec de la bromoéthylamine.

Le produit ainsi obtenu est parfaitement pur, soluble en milieu aqueux, apte à devenir insoluble après déshydratation, tout en étant suffisamment filmogène pour donner sans additif, des films dont les propriété physiques sont proches de celles d'une couche cornée normale tout en étant cytocompatible et biodégradable "in vivo".

Compte-tenu des caractéristique du produit, un problème important que se propose de résoudre l'invention, est de pouvoir réaliser un support artificiel faisant office de pansement et apte à reproduire de façon temporaire, certaines fonctions de l'épiderme.

Un tel problème est résolu en ce que l'on soumet les S-aminoéthylkératines en solution, à une opération de déshydratation pour obtenir un produit sous forme d'un film ou membrane homogène.

Le problème que se propose de résoudre l'invention est de créer un support artificiel sous forme d'une membrane notamment, présentant des caractéristiques physico-chimiques, semblables à celles de l'épiderme naturel ou de la couche cornée, à savoir, pour l'essentiel :

– une permabilité sélective à l'eau et aux petites molécules hydrosolubles,

– une taille des pores déterminée pour que la membrane soit à la fois imperméable aux molécules protéiques, à l'eau liquide, et aux agents infectieux et perméable à la vapeur d'eau, aux gaz et aux petites molécules hydrosolubles.

Une résistance à la rupture et une élasticité permettent de supporter les contraintes mécaniques imposées par l'environnement.

Un autre problème que se propose de résoudre l'invention, compte-tenu des caractéristiques de la membrane obtenue selon l'invention par déshydratation d'une solution de S-aminoéthylkératines, est d'obtenir une parfaite adhérence sur les tissus vivants, et, par conséquent, sur les plaies.

Pour résoudre de tels problèmes, la déshydratation de la solution des S-aminoéthylkératines, est effectuée de manière suffisamment lente pour permettre un arrangement précis des molécules donnant lieu à la formation de liaisons non covalentes, telles que liaisons électrostatiques, liaisons hydrogène, liaisons de Van Der Waals. De telles liaisons confèrent à la membrane, une résistance à la traction suffisante, compte-tenu des applications envisagées.

En outre, les microfibrilles de kératines sont conformées en hélice alpha et disposées sous forme de filaments, ce qui a pour effet de donner à la membrane une certiane élasticité.

L'arrangement moléculaire permet d'obtenir une membrane à structure poreuse, dont la taille des pores est fonction de la densité des liaisons intermoléculaires. Pour rendre les membranes insolubles dans leur milieu d'origine et dans les liquides physiologiques, les liaisons formées lors de la déshydratation de la solution des S-aminoéthylkératines, sont suffisamment fortes et la densité suffisamment élevée.

Ces liaisons sont majoritairement de nature électrostatiques et s'établissent entre les groupements carboxyliques présents à l'origine sur les microfibrilles de kératines natives sous forme de résidus acide glutamique et acide aspartique notamment, et les groupements amine présents dans les kératines natives sous forme de résidus lysile notamment et les groupements amine introduits par voie chimique sous forme de résidus aminoéthyle.

L'ensemble des phénomènes qui apparaissent au moment de la déshydratation provoquent la formation d'une structure tridimensionnelle de poids moléculaire élevé et qui est à l'origine du caractère insoluble précité. Les molécules utilisées pour la préparation des membranes sont biodégradables, tandis que leur association ne nécessite pas la mise en oeuvre d'un procédé externe de réticulation par voie chimique. Il en résulte que les membranes en tant que telles, présentent le caractère de biodégradabilité.

Les membranes de S-aminoéthylkératines obtenues peuvent présenter des dimensions variables en fonction de la taille des moules utilisés lors du processus de déshydratation et des épaisseurs variables suivant la quantité de solution mise en oeuvre et la concentration de ces solutions en S-aminoéthylkératines. Ces épaisseurs peuvent varier de quelques micromètres (2-20) à 1 ou 2 millimètres. Par exemple, les membranes de 50 à 150 micromètres d'épaisseur sont obtenues classiquement par le procédé de déshydratation à partir de solutions contenant entre 5 et 15 grammes de S-aminoéthylkératines par litre de solution. De telles membranes présentent des épaisseurs suffisantes pour obtenir des caractéristiques mécaniques compatibles avec leur utilisation comme moyen de protection des plaies.

Les membranes obtenues à l'état sec sont rigides et transparentes et deviennent souples et élastiques tout en gardant leur transparence lorsqu'elles sont réhydratées dans de l'eau pure ou dans des liquides physiologiques. Sous forme humide, ces membranes sont imperméables à l'eau liquide et perméable à la vapeur d'eau. A titre d'exemple, dans des conditions définies par l'expérience, une membrane de S-aminoéthylkératines de 100 micromètres d'épaisseur présente une perméabilité à la vapeur d'eau à 37 ° C et dans une atmosphère à 40 % d'humidité relative de 40 mg/cm2 h.

Sous forme humide, les membranes de kératines soumises à des tests de dialyse montrent une perméabilité aux molécules neutres qui est fonction de la masse moléculaire moyenne de ces dernières. A titre d'exemple, le seuil de coupure de membranes de 150 micromètres d'épaisseur est estimé à 2000 D, lorsque les tests de perméabilité sont effectués à l'aide de polyéthylèneglycols. Un tel seuil de coupure est incompatible avec le passage des protéines et des agents infectieux à travers ces membranes. En revanche, les molécules telles que sels, sucres, acides aminés, polysaccharides et peptides de faibles poids moléculaires, antibiotiques, par exemple, diffusent à travers les membranes.

Il convient maintenant d'analyser le procédé de préparation du produit à base de kératines, quelles que soient ses applications. Les principales étapes pour la préparation de la solution des S-aminoéthylkératines sont les suivantes :

– réduction des kératines,
– extraction des kératines ainsi réduites,
– alkylation de ces kératines réduites,
– extraction et purification des S-amino-éthylkératines.

Ces réactions successives sont réalisées par exemple sur de la laine, des poils d'animaux, des cheveux humains...

## REDUCTION DES KERATINES

Les kératines brutes sous forme de phanères sont broyées et mises en suspension dans une solution tamponnée ou non, maintenues à pH basique et contenant un agent réducteur doux. Le mélange hétérogène est maintenu sous agitation à température ambiante et à l'abri de l'air pendant 24 à 72 heures.

Divers mélanges tampons peuvent être utilisés tels que, à titre d'exemple : tris/HCl, NH3/NH4+, acide borique/borate, PO4⁻⁻/ HPO4⁻..., à condition que les tampons utilisés permettent de maintenir le pH du milieu entre pH 9 et pH 11,5.

Lorsqu'un mélange tampon n'est pas utilisé, le milieu peut être maintenu entre ces valeurs par addition de molécules basiques telles que par exemple, les hydroxydes alcalins ou alcalino-tereux, ou l'ammoniaque.

En ce qui concerne les agents réducteurs, le mercaptoéthanol est utilisé préférentiellement, mais d'autres molécules conviennent également telles que l'acide thioglycolique ou la cystéine par exemple.

## EXTRACTION DES KERATINES

Les kératines réduites sont recueillies en solution dans le liquide surnageant de centrifugation du milieu de réduction, le culot est éventuellement lavé une fois par la solution tampon et le liquide de lavage est ajouté au surnageant précédent.

Les kératines réduites sont préférentiellement précipitées à partir de la solution obtenue sous forme de microfibrilles à un pH compris entre 3,5 et 5, par addition d'un acide minéral concentré, tel que l'acide chlorhydrique 6N.

Le précipité est recueilli par centrifugation, filtration ou décantation et peut être rincé par une solution acide dont le pH est compris entre les valeurs précitées.

## ALKYLATION DES KERATINES REDUITES

On remet le précipité en solution à température ambiante et à l'abri de l'air dans un milieu basique tamponné ou non contenant de l'urée à une concentration comprise entre 3 et 6 M, de l'EDTA à une concentration d'environ $10^{-3}$ M et du mercaptoéthanol à une concentration comprise entre $2.10^{-2}$ et $5.10^{-2}$ M. Le pH du milieu est maintenu entre 9 et 11,5 pendant toute la durée de la dissolution par addition d'une base minérale concentrée telle que l'hydroxyde de sodium.

On prépare une solution de bromoéthylamine à 50 % en poids dans l'urée à une concentration comprise entre 3 et 6 M que l'on amène à pH neutre à l'aide par exemple, d'une solution d'hydroxyde de sodium 5 M. La masse de chlorhydrate de bromoéthylamine mise en solution est sensiblement égale à la masse de phanère mise en jeu dans l'étape de réduction.

On ajoute en une seule fois cette solution de bromoéthylamine à la solution de kératines réduites précédente tout en maintenant le pH du milieu réactionnel entre 8 et 9, par addition d'une solution concentrée d'hydroxyde de sodium, par exemple.
On agite le milieu obtenu, à l'abri de l'air et à température ambiante jusqu'à disparition totale des groupements thiol libres (test au nitroprussiate de sodium en milieu ammoniacal).

## EXTRACTION ET PURIFICATION DES S-AMINOETHYLKERATINES

On précipite les S-aminoéthylkératines à partir du milieu réactionnel précédent par 2,5 à 5 volumes d'acétone.

Après décantation, le précipité est collecté par toute technique de séparation solide-liquide connue en soi, telle que centrifugation ou filtration par exemple.

On dissout le précipité obtenu dans environ 20 à 30 fois son volume d'une solution aqueuse d'acide acétique maintenue à pH 3 - 3,7. Les résidus insolubles sont éliminés par centrifugation ou filtration.

On ajoute au milieu clarifié obtenu, une solution concentrée de chlorure de potassium en quantité suffisante pour obtenir une concentration finale en kCl d'environ 0,2 M puis on précipite les S-aminoéthylkératines en augmentant le pH jusqu'à environ 9 - 10 par addition d'une solution d'hydroxyde acalin concentrée.

On reprend le précipité obtenu dans environ 15 à 20 fois son volume d'une solution d'acide acétique maintenue à pH compris entre 3 et 3,7.

On peut renouveler l'opération de précipitation et redissoudre le culot de 2ème précipitation dans 15 à 20 fois son volume d'une solution d'acide acétique maintenue à pH compris entre 3 et 3,7.

On dialyse la solution obtenue contre de l'eau distillée à l'aide de membranes dont le seuil de coupure est voisin de 10 KD.

On agit sur la solution dialysée pour éliminer les résidus non dissous, ladite solution étant conservée à une température de l'ordre de + 4° C.

Les solutions de S-aminoéthylkératines peuvent être concentrées en fonction des besoins par toute technique connue et en particulier par ultrafiltration sur membranes dont le seuil de coupure est voisin de 10 KD.

Le procédé tel que décrit dans ses principales étapes est applicable pour obtenir une solution de S-aminoéthylkératines à partir de laine, de poils d'animaux tels que lapin, rat, veau, porc... et de cheveux humains.

On donne ci-après un exemple de préparation d'une solution de S-aminoéthylkératines à partir de laine.

## REDUCTION DES KERATINES

– 100 g de laine sont mis en suspension dans sensiblement 1,8 l d'une solution aqueuse de Tris 0,01 M contenant 0,5 % de mercaptoéthanol. Le pH est ajusté à 10,5 avec une solution d'hydroxyde de sodium 5 M.
– Le milieu est broyé pour obtenir une suspension homogène, puis est placé sous agitation à température ambiante à l'abri de l'air pendant 24 à 72 heures.

## EXTRACTION DES KERATINES

– On centrifuge le milieu de réduction et on recueille le surnageant qui contient les kératines réduites. Le culot est lavé par 2 à 3 fois son volume de tampon Tris à 0,01 M à pH 10,5 et le liquide de lavage est mélangé au surnageant précédent.

– La solution obtenue est acidifiée jusqu'à pH 4,5 par addition d'acide chlorhydrique 6 N. Le précipité formé est recueilli par centrifugation.

## ALKYLATION DES KERATINES REDUITES

– On remet le précipité en solution dans 1,2 l de tampon Tris 0,01 M, urée 5 M, EDTA 0,001 M contenant 2 ml de mercaptoéthanol.

– On ajuste le pH à 10,5 avec une solution d'hydroxyde de sodium 5 N. On maintient ledit pH à cette valeur pendant toute la durée de la dissolution,

– On prépare une solution contenant 100 g de chlorhydrate de bromoéthylamine dans 75 ml d'urée 5 M que l'on amène à pH 7 à l'aide d'une solution d'hydroxyde de sodium 5 N,

– On ajoute cette solution à la solution de kératines réduites toute en maintenant le pH entre 8,0 et 9,0 avec une solution d'hydroxyde de sodium 5 N,

– On agite le milieu obtenu à l'abri de l'air à température ambiante, en contrôlant le pH dont la valeur doit rester comprise entre 8,5 et 9,0,

– La réaction est terminée lorsque la recherche des fonctions thiol par le test au nitroprussiate de sodium est négative.

## EXTRACTION ET PURIFICATION DES S-AMINOETHYLKERATINES

– On précipite les S-aminoéthylkératines à partir de la solution obtenue dans l'étape précédente par 2,5 volumes d'acétone. Après décantation, le précipité est recueilli par centrifugation,

– On redissout le culot dans 3,5 l d'eau distillée tout en maintenant le pH à environ 3,5 par addition d'acide acétique pur,

– On ajoute à la solution obtenue une solution saturée de chlorure de potassium pour obtenir une concentration finale de 0,2 M puis on précipite les S-aminoéthylkératines à pH 9,0 par addition d'une solution d'hydroxyde de sodium environ 5 N,

– On recueille le précipité formé par centrifugation après décantation,

– On dissout le culot obtenu, dans 2,5 l d'eau distillée, tout en mantenant le pH à environ 3,5 par addition d'acide acétique pur,

– On renouvelle l'opération de précipitation et on redissout le culot dans 2,5 l d'une solution d'acide acétique maintenue à pH 3,5.

Cette solution de S-aminoéthylkératines est ensuite soumise à un certain type de traitement, pour obtenir différents types de produits, compte-tenu des applications possibles.

Comme indiqué en liminaire, si l'on soumet les solutions de S-aminoéthylkératines à une opération de déshydratation, le produit obtenu se présente sous forme d'un film ou d'une membrane homogène. A titre d'exemple indicatif, les membranes sont obtenues par déshydratation d'une solution contenant 5 à 15 mg de S-aminoéthylkératines par ml.

La solution est préalablement filtrée ou centrifugée pour éliminer d'éventuels insolubles et dégazée. La déshydratation peut être effectuée à des températures de l'ordre de 40 à 42° C.

Il est évident que la déshydratation peut être effectuée à différentes températures en fonction du produit désiré. Par exemple, une déshydratation rapide peut être obtenue à 60° C sans dégradation notable du produit. La température ne doit cependant pas excéder 90° C si l'on veut obtenir des membranes homogènes. Au delà de cette température, des dégradations apparaissent objectivées par la formation de petits peptides.

Les membranes obtenues peuvent être neutralisées par trempage dans un tampon borate à un pH d'environ 9,0 - 9,5 puis rincées à l'eau distillée. Ces membranes peuvent être ensuite déshydratées dans de l'alcool par exemple, ou sous courant d'air pour assurer leur conservation.

A noter que les membranes peuvent être cependant conservées à l'état humide dans de l'eau ou dans un mélange alcool-eau.

Les films ou membranes obtenus par la solution de S-aminoéthylkératines trouvent de nombreuses applications, notamment en médecine humaine ou vétérinaire, parmi lesquelles on peut noter :

– recouvrement des plaies
– support de culture cellulaire
– transfert de greffes
– étude de la toxicité et de la diffusion des molécules en dermatologie
– support pour le greffage d'enzymes et de cellules.

Lorsque l'on soumet les S-aminoéthylkératines à une opération de lyophilisation, on obtient un produit sous forme d'un feutre dont le diamètre des pores est modulable en fonction des conditions opératoires. Parmi les principales applications, on peut citer la réalisation d'un réseau tridimensionnel comme support de cellules vivantes.

Lorsque l'on soumet les S-aminoéthylkératines à une opération de déshydratation, d'une part, et de broyage, d'autre part, on obtient un produit sous forme de poudres, de granulométrie variable. Ces poudres peuvent être utilisées en tant que support de chromatographie, et trouvent également des applications en cosmétique.

Lorsque l'on soumet les S-aminoéthylkératines à des techniques diphasiques classiques, on obtient un produit sous forme de sphérules poreuses. Ces sphérules peuvent être utilisées en tant que support cellulaire et de chromatographie, ainsi qu'en support de greffage de cellules et d'enzymes.

Lorsque l'on soumet les S-aminoéthylkératines à une opération de coagulation ou ou coagulation - extrusion et de séchage, on obtient un produit sous forme de fils ou de fibres. Ces fils et fibres peuvent constituer des fils de suture biodégradables ou des tissus biodégradables, telles que les gazes.

A noter également que la solution S-aminoéthylkératines peut être utilisée directement pour l'imprégnation de matériaux poreux et le recouvrement de matériaux poreux ou non, par exemple.

Enfin, ces différentes formes de produits, peuvent trouver des applications en cosmétologie, telles que par exemple :

– agents exfolliants (dans le cas de poudres et de sphérules)

– agents de surface (dans le cas de solutions et de poudres)

– masques (dans le cas de solutions, de poudres et de films)

– additifs capillaires (dans le cas de solutions et de poudres).

**Revendications**

-1- Produit à base de kératines modifiées, caractérisé en ce qu'il s'agit d'une S-aminoAlkylkératines de fomule générale :

$$R - S - (CH2) n - NH2$$

dans laquelle :

– n est un nombre entier égale à 2 ou 3

– R représente la kératine

-2- Produit selon la revendication 1, caractérisé en ce qu'il est obtenu à partir d'une solution de S-aminoéthylkératines de formule :

$$R-S-CH2-CH2-NH2$$

obtenues par réduction puis S-alkylation des kératines réduites.

-3- Procédé de préparation du produit selon l'une quelconque des revendications 1 et 2, caractérisé en ce que :

– on réduit les kératines

– puis on extrait les kératines ainsi réduites

– puis on opère l'alkylation de ces kératines réduites

– et enfin, on extrait et on purifie les S-aminoéthylkératines

-4- Procédé selon la revendication 3, caractérisé en ce que pour la réduction des kératines:

– on broie les kératines brutes et on les met en suspension dans une solution contenant un agent réducteur.

– on maintient le mélange hétérogène sous agitation à température ambiante et à l'abri de l'air pendant une durée déterminée

-5- Procédé selon la revendication 3, caractérisé en ce que pour l'extraction des kératines réduites :

– on recueille lesdites kératines en solution dans le liquide surnageant, du milieu de réduction, le culot étant éventuellement lavé par la solution contenant l'agent réducteur, le liquide de lavage étant ajouté au surnageant.

– on précipite les kératines

– on recueille le précipité obtenu.

-6- Procédé selon l'une quelconque des revendications 3 et 5, caractérisé en ce que pour l'alkylation des kératines réduites :

– on remet le précipité en solution à température ambiante et à l'abri de l'air dans un milieu basique.

– on rajoute une quantité de réducteur

– on maintient le pH du milieu entre 9,0 et 11,5 pendant toute la durée de la dissolution

– on prépare une solution de Bromoéthylamine que l'on amène à pH neutre.

– on ajoute en une seule fois, la solution de Bromoéthylamine à la solution de kératines réduites précédente tout en maintenant le pH entre 8,0 et 9,0.

– on agite le milieu obtenu à l'abri de l'air et à température ambiante.

-7- Procédé selon l'une quelconque des revendications 3, 4, 5 et 6, caractérisé en ce que pour l'extraction et la purification des S-aminoéthylkératines :

– on précipite lesdites S-aminoéthylkératines à partir de la solution

– après décantation, on recueille le précipité

– on dissout le précipité obtenu pour maintenir le pH à 3,0 - 3,7

– on élimine les résidus insolubles

– on ajoute une solution de chlorure de potatium notamment, pour obtenir une concentration finale en KCl d'environ 0,2M, puis on précipite les S-aminoéthylkératines en augmentant le ph jusqu'à sensiblement 9,0 - 10.

– on reprend le précipité obtenu dans environ 15 à 20 fois son volume dans une solution maintenue à pH compris entre 3,0 et 3,7

– on dialyse la solution obtenue contre de l'eau distillée

– on agit sur la solution dialysée pour éliminer les résidus non dissous, ladite solution étant conservée à une température de l'ordre de + 4° C.

-8- Procédé selon l'une quelconque des revendications 3 à 7, caractérisé en ce que l'on soumet les S-aminoéthylkératines à une opération de déshydratation pour obtenir un produit sous forme d'un film ou membrane homogène.

-9- Procédé selon l'une quelconque des revendications 3 à 7, caractérisé en ce que l'on soumet les S-aminoéthylkératines à une opération de lyophilisation pour obtenir un produit sous forme d'un feutre dont le diamètre des pores est modulable en fonction des conditions opératoires.

**-10-** Procédé selon l'une quelconque des revendications 3 à 7, caractérisé en ce que l'on soumet les S-aminoéthylkératines à une opération de déshydratation et de broyage pour obtenir un produit sous forme de poudres de granulométries variables.

**-11-** Procédé selon l'une quelconque des revendications 3 à 7, caractérisé en ce que l'on soumet les S-aminoéthylkératines à une opération de techniques diphasiques pour obtenir un produit sous forme de sphérules poreuses.

**-12-** Procédé selon l'une quelconque des revendications 3 à 7, caractérisé en ce que l'on soumet les S-aminoéthylkératines à une opération de coagulation ou coagulation - extrusion et séchage pour obtenir un produit sous forme de fils ou de fibres.

**-13-** Applications des S-aminoéthylkératines sous forme de films ou membranes selon la revendication 9 :

– au recouvrement des plaies
– à un support de culture cellulaire
– à un transfert de greffes
– à l'étude de la toxicité et de la diffusion des molécules en dermatologie
– à un support pour le greffage d'enzymes et de cellules.

**-14-** Applications des S-aminoéthylkératines sous forme d'un feutre selon la revendication 10 à un réseau tridimensionnel utilisé comme support de cellules vivantes.

**-15-** Applications des S-aminoéthylkératines sous forme de poudres, selon la revendication 11 à un support de chromatographie et à des utilisations cosmétiques.

**-16** Applications des S-aminoéthylkératines sous forme de sphérules selon la revendication 12 :

– à un support de culture cellulaire et de chromatographie
– à un support de greffage de cellules et d'enzymes

**-17-** Applications des S-aminoéthylkératines sous forme de fils ou fibres selon la revendication 13 à des fils de suture biodégradables ou tissus biodégradables.

Office européen
des brevets

RAPPORT DE RECHERCHE EUROPEENNE

Numero de la demande

EP 91 42 0138

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | KOBUNSHI RONBUNSHU, vol. 39, no. 4, 1982, pages 221-227, Tokyo, JP; NOISHIKI et al.: "Application of denatured wool keratin derivatives to an antithrombogenic siomaterial-vascular graft coated with a heparinized kerotin derivative" * Page 221, section 2.1; page 223, tableau 1; page 227 * | 1 | C 07 K 15/20<br>A 61 K 37/12<br>C 12 N 5/00<br>A 61 L 15/32 |
| Y | EP-A-0 243 151 (AJINOMOTO) * Page 4, ligne 9 - page 5, ligne 19 * | 1-17 | |
| Y,D | FR-A-2 529 214 (L'OREAL) * Pages 1-7 * | 1-17 | |
| Y,D | FR-A-2 522 657 (L'OREAL) * Pages 1-5 * | 1-17 | |
| | | | DOMAINES TECHNIQUES RECHERCHES (Int. Cl.5) |
| | | | C 07 K<br>A 61 K<br>C 12 N<br>A 61 L |

Le présent rapport a été établi pour toutes les revendications

| Lieu de la recherche | Date d'achèvement de la recherche | Examinateur |
|---|---|---|
| LA HAYE | 12-07-1991 | KORSNER S.E. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant